# EUROPÄISCHE PATENTSCHRIFT

(11) **EP 3 099 714 B1**
(45) Veröffentlichungstag und Bekanntmachung des Hinweises auf die Patenterteilung: **28.04.2021**
(21) Anmeldenummer: 15713829.8
(22) Anmeldetag: 23.01.2015
(51) Int. Cl.: C07K 16/24

(54) **HYBRIDOM-ZELLLINIE (MY-C-CC0C2-259-1 A4) UND DEREN VERWENDUNG ZUR HERSTELLUNG EINES MONOKLONALEN ANTIKÖRPERS GEGEN DAS HUMANE, HERZSPEZIFISCHE MYOSIN BINDING PROTEIN C (C-PROTEIN, MYBPC3, CMYBP-C ODER MY-C)**
HYBRIDOMA CELL LINES (MY-C-CC0C2-259-1 A4) AND USE THEREOF FOR PRODUCING A MONOCLONAL ANTIBODY AGAINST HUMAN CARDIAC MYOSIN BINDING PROTEIN C (C-PROTEIN, MYBPC3, CMYBP-C OR MY-C)
HYBRIDOME (MY-C-CC0C2-259-1 A4) ET SON UTILISATION POUR PRODUIRE UN ANTICORPS MONOCLONAL DE LUTTE CONTRE LA PROTÉINE C HUMAINE SPÉCIFIQUE DU COEUR, LIANT LA MYOSINE (PROTÉINE C, MYBPC3, CMYBP-C OU MY-C)

(30) Priorität: 27.01.2014 DE 102014000856
(43) Veröffentlichungstag der Anmeldung: 07.12.2016
(73) Patentinhaber: King's College London, London WC2R 2LS (GB)
(72) Erfinder: WEBER, Ekkehard, 06120 Halle (Saale) (DE); MEDEK, Rita, 06108 Halle (Saale) (DE)
(74) Vertreter: Wilson, Justin Scott
(86) Internationale Anmeldenummer: PCT/DE2015/000028
(87) Internationale Veröffentlichungsnummer: WO 2015/110114

(56) Entgegenhaltungen:
- EP-A1- 0 435 185
- WO-A1-2008/104289
- Jeanne Theis: "Expression Patterns of Cardiac Myofilament Proteins", , 13. Mai 2009 (2009-05-13), Seiten 325-333, XP055197660, Gefunden im Internet: URL:http://circheartfailure.ahajournals.or g/content/2/4/325 [gefunden am 2015-06-23]
- Keiko Saruta ET AL: "Differential Expression of Two Cardiac Myosin-Binding Protein-C Isoforms in Developing Chicken Cardiac and Skeletal Muscle Cells", ZOOLOGICAL SCIENCE., vol. 27, no. 1, 1 January 2010 (2010-01-01), pages 1-7, XP055603541, JP ISSN: 0289-0003, DOI: 10.2108/zsj.27.1

## Beschreibung

Die Erfindung betrifft einen Maus-Hybridom-Klon, der einen monoklonalen Antikörper (anti-My-C-cC0C2-259-1A4; IgG1, kappa) produziert, der gegen das kardiale Myosin Binding Protein C (C-protein, MYBPC3, cMyBP-C oder My-C) gerichtet ist und es detektiert und der nicht mit den nahe verwandten Isomeren von My-C aus der Skelettmuskulatur reagiert. Dieser mAK eignet sich als Catcher- oder als Detektor-Antikörper für den Aufbau eines ELISA (Enzyme-linked Immuno Sorbent Assay) zur quantitativen Bestimmung von My-C in Serum, Plasma, Vollblut oder anderen Körperflüssigkeiten für die frühe Diagnostik von Herzinfarkten. Im Rahmen dieses diagnostischen Verfahrens kann er eine deutlich frühere Therapie von Myokardinfarkten ermöglichen.

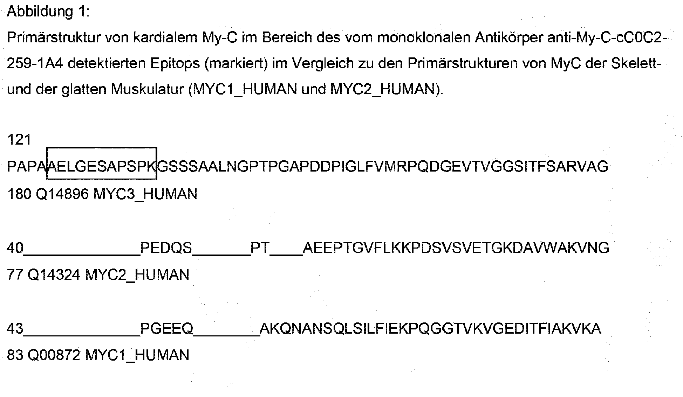

Wegen der akuten Lebensgefahr müssen Myokardinfarkte schnell diagnostiziert und von anderen Ursachen für Brustkorbschmerzen unterschieden werden. [1]
Die Bestimmung von Biomarkern für Herzmuskelnekrosen ist inzwischen essentiell für die Diagnostik von Infarkten bei vermuteten NSTE-ACSs (non-ST elevation acute coronary syndromes) und zwingend für die Diagnosestellung im entsprechenden klinischen Zusammenhang. Die kardialen Troponine (cTn) gelten im Moment als die entscheidenden Biomarker. Sie sind Bestandteil der allgemeinen Infarkt-Definition. [2] Die kardialen Troponine (cTn) haben aber Defizite und neue Biomarker könnten sich als sehr wertvoll erweisen. [3]
Die cTn-Konzentration im Serum erreicht erst 16-18 Stunden nach Einsetzen der Symptome das Maximum und ein Nachteil der bisherigen cTn-Tests ist ihre mangelnde analytische Sensitivität, um die niedrigen cTn-Konzentrationen in den ersten Stunden nach Beginn der Symptomatik zu erfassen. [4; 5] Neuere cTn-Tests streben die verlässliche Bestimmung niedriger cTn-Werte an, aber ihre reduzierte Spezifität gegenüber Infarkten mindert ihren Wert, weil cTn-Konzentrationen in der Nähe der 99-Perzentile gesunder Probanden zu beachten sind. Aber selbst dann liegen die cTn-Konzentrationen bei bis zu 25% der Infarkt-Patienten unterhalb dieser Schwelle. [6]
In Anbetracht der begrenzten Sensitivität und Spezifität der cTn-Tests wird in entsprechenden Guidelines (NICE) empfohlen, 10-12 Stunden nach Beginn der Symptome (Schmerzen im Bereich des Thorax) die cTn zu bestimmen, um die Diagnose zu sichern. [1] Obwohl es eine Reihe von Biomarkern gibt, die nach einem Infarkt schneller freigesetzt werden, hat sich keiner von ihnen durchsetzen können, weil sie nicht herzspezifisch exprimiert werden. [7] Aus diesem Grunde konzentrieren sich gegenwärtig die Bemühungen auf die Analyse des Ausmaßes der zeitlichen Änderungen der cTn-Konzentrationen, um die Aussagefähigkeit der cTn-Tests zu verbessern. Dabei bleibt unklar, wie groß der absolute Konzentrationsunterschied sein müsste, um Unterschiede in der analytischen und biologischen Variation der cTn-Konzentrationen für die beabsichtigte Diagnosestellung bedeutungslos werden zu lassen.
Der ideale Biomarker müsste schnell aus dem Myocard nach einem Infarkt freigesetzt werden, aber - im Gegensatz zu vergleichbaren bisherigen Markern - herzspezifisch sein. Das kardiale Myosin Binding Protein C (C-protein, MYBPC3, cMyBP-C oder My-C) ist ein Protein, das diese Kriterien erfüllt. Es wurde bei der Proteom-Analyse des koronaren Effluxes ischämischer Mausherzen identifiziert. [8] Es gehört zu den am höchsten im Myokard exprimierten Proteinen (19. unter 2.300 Proteinen) und ist zumindest doppelt so konzentriert wie cTnl and cTnT (92., bzw. 118. unter 2.300 Proteinen). [9]
Es existieren 3 verschiedene My-C-Isomere, die durch unterschiedliche Gene codiert werden. Die herzspezifische Isoform hat im Unterschied zu My-C der schnellen und des My-C der langsamen Skelettmuskulatur eine einzigartige N-terminale Domäne (Abb. 1) und andere herzspezifische Bereiche, die als spezifische Epitope dienen könnten. [10]
Die Freisetzung von My-C nach Myokardinfarkten bzw. -schäden wurde nachgewiesen [8; 11; 12; 13; 14] und der zeitliche Verlauf der Konzentrationsanstiege mit denen von cTn verglichen. EP 0 435 185 offenbart monoklonale Antikörper, die myocardiales C Protein binden.
Der Erfindung lag die Aufgabe zugrunde, in vitro produzierbare monoklonale Antikörper gegen herzspezifische Epitope des humanen My-C durch die Generierung von Myelomzellklonen herzustellen, die solche spezifischen Antikörper mit Epitopspezifität produzieren. Diese monoklonalen Antikörper sollten u.a. den Aufbau eines ELISA (Enzyme-Linked Immuno Sorbent Assay) zur spezifischen, kreuzreaktivitätsfreien quantitativen Bestimmung von My-C in Serum, Plasma oder Vollblut ermöglichen.
Die Aufgabe wird durch Generierung eines Hybridomzellklons gelöst, der einen monoklonalen Antikörper produziert, der ein herzspezifisches Epitop im My-C erkennt und bindet und der keine Kreuzreaktivität gegenüber den Myosin Binding Proteins der Skelettmuskulatur aufweist. Die Hybridomzelllinie ist durch Fusion von Myelomzellen mit Milzzellen eines gegen rekombinantes My-C immunisierten Versuchstieres, insbesondere der Maus, erhältlich. Die Hybridomzelllinie wurde am 10. Dezember 2013 gemäß den Erfordernissen des Budapester Vertrages bei der DSMZ mit der Eingangsnummer DSM ACC3224 hinterlegt. Der von diesem Hybridomzellklon produzierte Antikörper eignet sich in Kombination mit einem oder mehreren anderen mAK in einem ELISA zur sensitiven

Bestimmung der Konzentration von My-C im Serum und damit für die frühzeitige Diagnose von Herzinfarkten.

Weiterhin ist Gegenstand der Erfindung der von der Hybridomzelllinie produzierte epitopspezifische Antikörper und dessen Verwendung.

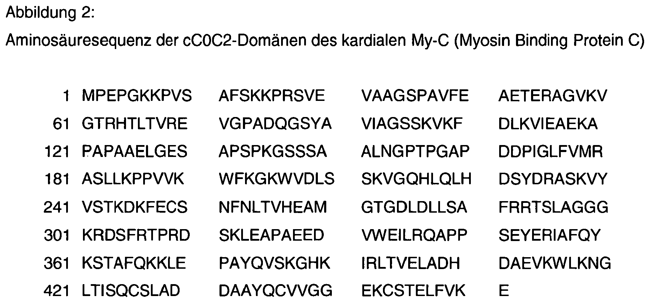

Zur Generierung von Hybridomklonen, die monoklonale Antikörper gegen humanes, herzspezifisches My-C produzieren, wurden Balb/c-Mäuse in bekannter Weise im Abstand von sechs bis acht Wochen mit den rekombinanten Domänen cC0C2 des My-C (Abb. 2) immunisiert. Die Mäuse werden vor der Entnahme der Milz geboostert. Die isolierten Milzzellen werden in bekannterweise mit Zellen der Maus-Myelomzellinie P3X63Ag8.653 (ATCC CPL 1580) fusioniert und in geeigneten Medien kultivert. [15] Die Hybridome, die ausschließlich Antikörper gegen das humane My-C produzieren, werden selektiert, mehrfach kloniert und propagiert. Die primäre Selektion dieser spezifischen Hybridome wurde mit Hilfe eines ELISA durchgeführt, bei dem das C0C2-Peptid des My-C an die Oberfläche von Mikrotiterplatten adsorbiert wurde.

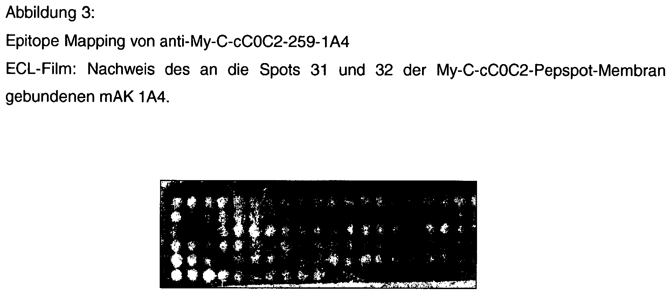

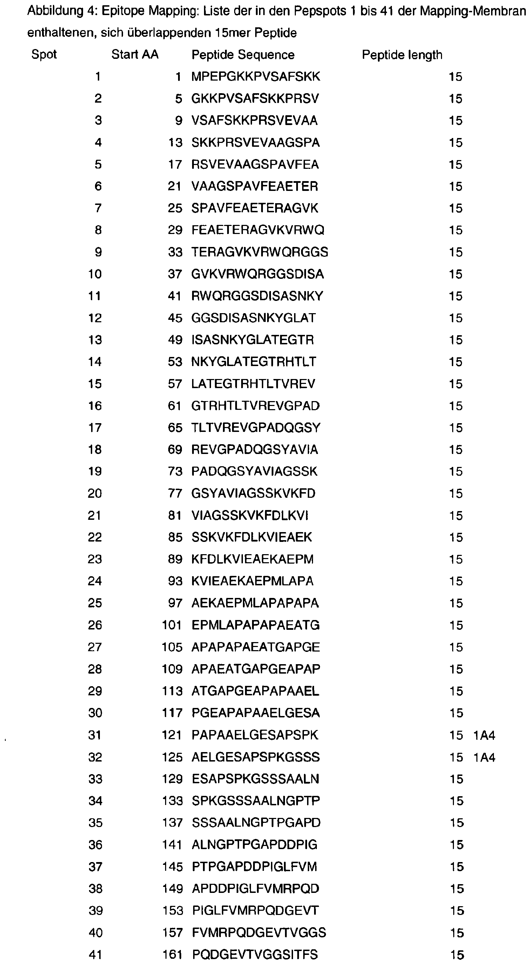

Die Epitopspezifität des monoklonalen Antikörpers des aus den Klonen nach diesen Kriterien selektierten erfindungsgemäßen Klons wurde durch Peptid-Scanning (Pepscan) ermittelt (16, 17, 18). Dazu wurden Peptide (sequenzidentisch mit der für die Immunisierung eingesetzten cC0C2-Domäne des My-C) mit einer Länge von 15 Aminosäureresten als einzelne Spots auf einer Membran synthetisiert. Die Sequenzen der 15mer-Peptide benachbarter Spots überlappten sich, so dass die gesamte Aminosäuresequenz der cC0C2-Domäne des My-C in insgesamt 111 Spots überlappend synthetisiert war. Diese Peptide wurden auf der Mapping-Membran mit dem erfindungsgemäßen monoklonalen Antikörper inkubiert. Der Nachweis des gebundenen Antikörpers erfolgte mit dem ECL™ (Enhanced Chemiluminescence) - System auf Film.
Mit Hilfe dieses Verfahrens konnte ermittelt werden, welche der 15mer Peptide von dem erfindungsgemäß hergestellten monoklonalen Antikörper erkannt werden. Über die bekannte Sequenz der Peptide in den detektierten Einzelspots (vgl. Abb. 3) ließ sich auf die Aminosäuresequenz des durch den monoklonalen Antikörper des Hybridom-Klons erkannten Epitopes des humanen My-C schließen. (Abb. 4)

Der durch den erfindungsgemäß erzeugten Hybridom-Klon produzierte monoklonale Antikörper 1A4 bindet im humanen My-C an das Epitop mit der Sequenz
-A125 -E-L-G-E-S-A-P-S-P-K-

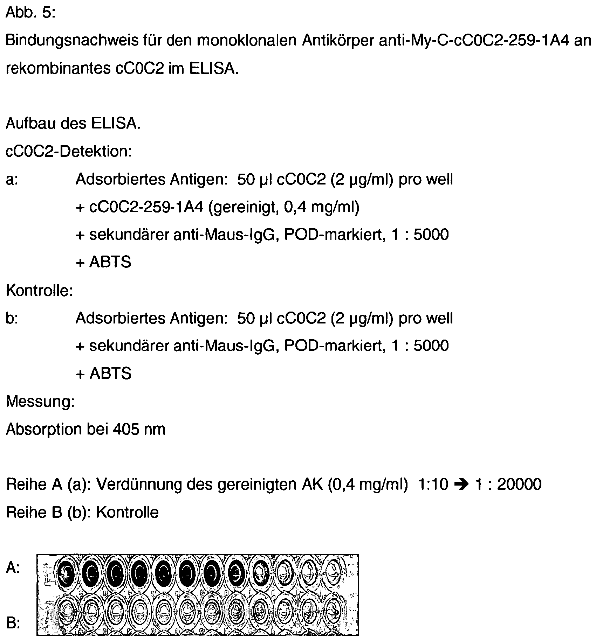

Der Nachweis, dass der erfindungsgemäß hergestellte monoklonale Antikörper nicht nur Peptide auf dieser Pepspot-Membran detektiert, sondern auch das gesamte Molekül der cC0C2-Domäne des humanen My-C, das dieses Epitop enthält, wurde durch seinen Einsatz im ELISA gezeigt. Ein solcher beispielhafter ELISA mit dem monoklonalen Antikörper 1A4 ist in Abbildung 5 gezeigt.
Der oben aufgeführte, epitopcharakterisierte monoklonale Antikörper (IgG₁, kappa) kann in seiner nativen Form oder als Fragment modifiziert oder markiert werden. Dieser Antikörper oder seine modifizierten Formen sind einsetzbar für die Aufklärung des Processings des humanen My-C, der Kinetik seiner Freisetzung und seiner Clearance aus dem Serum, für dessen qualitativen Nachweis und seine quantitative Bestimmung (z.B. ELISA und Western Blot), in der Immunhistologie oder als Diagnostikum,

Die Erfindung wird nachfolgend durch Ausführungsbeispiele näher erläutert.

### Ausführungsbeispiele

### Beispiel 1: Herstellung der Hybridomzelllinie

Die Milz einer in bekannter Weise mit cC0C2 des My-C immunisierten Maus wird unter sterilen Bedingungen entnommen und die Milzzellen werden mit RPMI1640-Medium (Life Technologies™, Karlsruhe) mit einer Spritze aus der Milzkapsel heraus gespült und vereinzelt. Die Milzzellen werden pelletiert (10 Minuten bei 300 x g), dreimal mit RPMI1640-Medium gewaschen und in RPMI1640-Medium resuspendiert. Sie werden dann mit Myelomzellen der Linie P3X63Ag8.653 (ATTC CPL 1580) fusioniert. Dafür werden kultivierte Myelomzellen, die sich in der log-Phase des Wachstums befinden, ebenfalls pelletiert und dreimal gewaschen. 1 x 10⁸ Milzzellen und 5 x 10⁷ Myelomzellen werden in ein Zentrifugenrohr pipettiert, intensiv gemischt und abzentrifugiert und zu dem Zell-Sediment werden innerhalb einer Minute 1,5 ml vorgewärmtes 50 %iges Polyethylenglykol 1500 (Roche, Basel) tropfenweise und unter kontinuierlichem Drehen des Röhrchens bei 37° C gegeben. Der Fusionsansatz wird dann eine weitere Minute bei 37° C inkubiert. In den folgenden drei Minuten wird vorgewärmtes Medium (RPMI1640) tropfenweise zugegeben - in der ersten Minute 1 ml, in der zweiten Minute 3 ml und dann 18 ml. Anschließend wird sofort bei 200 x g für 10 Minuten zentrifugiert. Das Zell-Pellet wird in RPMI1640-Medium mit 10% FCS und HAT aufgenommen. Ein Teil des Pellets wird in 96-well Kulturplatten ausgesät und der Rest in flüssigem Stickstoff bei - 196°C eingefroren. Als Feederzellen dienen bei der Kultivierung Maus-Peritoneal-Makrophagen, die 1 Tag vor der Fusion in Kultur genommen wurden (1 x 10⁴ Makrophagen pro well in HAT-Medium). Die Zellen werden in einem CO₂-Brutschrank bei 37° C inkubiert. Das Medium wird jeweils nach 3 - 5 Tagen durch frisches RPMI1640-HAT-Medium ersetzt und in Abhängigkeit vom Wachstum der fusionierten Zellen werden die Kulturüberstände nach ca. 2 Wochen mit einem ELISA auf ihre Reaktivität gegenüber dem Antigen (My-C) getestet.

### Beispiel 2: Selektion der Antikörper-produzierenden Klone

Alle aufwachsenden Klone, bzw. ihre Antikörper, wurden auf ihre Reaktivität mit Hilfe eines ELISA (Enzyme-Linked Immunosorbent Assay) getestet. Immunosorbent war das Immunogen, die rekombinante cC0C2-Domäne des My-C (ca. 2 µg/ml).
Durchführung des ELISA:
1. Beschichtung der Mikrotiterplatten (Costar, high binding) mit je 50 µl Immunogenlösung pro well bei 4° C über Nacht;
2. Waschen der Mikrotiterplatten (MTP), 3 mal mit TBS (TRIS-buffered saline), pH 7,4;
3. Blockierung der MTP mit 200 µl Blocking Reagent (Boehringer, Mannheim) pro well, 1 Stunde bei 37° C;
4. Waschen der MTP, 3 mal mit NaCI-Tween 20;
5. Inkubation mit Kulturüberstand der Hybridomkulturen; je 50 µl pro well, ca. 1 : 2 verdünnt mit TBS-Tween 20;
6. Waschen der MTP, 3 mal mit NaCI-Tween 20;
7. Inkubation mit anti-Maus-Ig-Antikörpern, gekoppelt an Peroxidase, 50 µl pro well, 1 Stunde bei Raumtemperatur;
8. Waschen der MTP, 3 mal mit NaCl-Tween 20;
9. Inkubation mit ABTS-Lösung (100 mg ABTS pro 100 ml Substrat-Puffer [Zitrat, Natriumperborat, pH 4,4]), 50 µl pro well;
10. Messung bei 405 nm nach 60 Minuten Inkubationszeit bei Raumtemperatur mit einem Microplate Reader (SLT).

### Beispiel 3: Epitop-Kartierung für den monoklonalen Antikörper 1A4 im humanen, herzspezifischen My-C

Die Identifizierung der Bindungsstelle des monoklonalen Antikörpers 1A4 erfolgte mit der Methode des Peptid-Scannings. Dabei wird die gesamte Aminosäure-Sequenz der humanen cC0C2-Domäne des My-C, das für die Immunisierung eingesetzt wurde, in insgesamt 111 sich überlappende Aminosäure-Sequenzen von jeweils 15 Aminosäuren Länge geteilt. Diese Sequenzen werden als einzelne Peptide in Spots direkt auf einer Cellulose-Membran synthetisiert. Die Membran wird mit den antikörperhaltigen Kulturüberständen der Hybridome inkubiert und die Bindungsstellen der Antikörper durch Inkubation mit einem Peroxidasegekoppelten Anti-Maus-Ig-Antikörper sichtbar gemacht. Dazu wird nach dreimaligem Waschen mit TBS-Tween die Membran zwischen Kopierfolien gelegt, dann mit dem ECL™ (Enhanced Chemiluminescent) Detection Reagent (Amersham, Braunschweig) 3 Minuten inkubiert. Ein aufgelegter Film (Hyperfilm-ECL™ [RPN 2103H Amersham, Braunschweig]) wird anschließend zwischen 30 Sekunden und 3 Minuten belichtet. Die Identifizierung der durch den Antikörper detektierten Sequenzen erfolgt durch Zuordnung der auf dem Film belichteten Spots 31 und 32 (Abb. 4) zu den in den Spots lokalisierten 15mer Teilsequenzen des Immunogens (cC0C2-Domäne des My-C).

| | | | | |
|---|---|---|---|---|
| Spot 31 | 121 | PAPAAELGESAPSPK | 15 | 1A4 |
| Spot 32 | 125 | AELGESAPSPKGSSS | 15 | 1A4 |

Die erkannte zentrale Sequenz der beiden Teilsequenzen ist die Aminosäure-Folge -A125-E-L-G-E-S-A-P-S-P-K-. Diese Sequenz ist das detektierte Epitop, an das der Antikörper 1A4 im humanen My-C bindet.

### Literatur

1. Cooper A, Timmis A, Skinner J. Assessment of recent onset chest pain or discomfort of suspected cardiac origin: Summary of nice guidance. BMJ. 2010;340:c1118
2. Thygesen K, Alpert JS, Jaffe AS, Simoons ML, Chaitman BR, White HD, Katus HA, Lindahl B, Morrow DA, Clemmensen PM, Johanson P, Hod H, Underwood R, Bax JJ, Bonow RO, Pinto F, Gibbons RJ, Fox KA, Atar D, Newby LK, Galvani M, Hamm CW, Uretsky BF, Steg PG, Wijns W, Bassand JP, Menasche P, Ravkilde J, Ohman EM, Antman EM, Wallentin LC, Armstrong PW, Januzzi JL, Nieminen MS, Gheorghiade M, Filippatos G, Luepker RV, Fort-mann SP, Rosamond WD, Levy D, Wood D, Smith SC, Hu D, Lopez-Sendon JL,Robertson RM, Weaver D, Tendera M, Bove AA, Parkhomenko AN, Vasilieva EJ, Mendis S. Third universal definition of myocardial infarction. Circulation. 2012;126:2020-2035
3. Gerszten RE, Carr SA, Sabatine M. Integration of proteomic-based tools for improved biomarkers of myocardial injury. Clin.Chem. 2010;56:194-201
4. Katus HA, Remppis A, Neumann FJ, Scheffold T, Diederich KW, Vinar G, Noe A, Matern G, Kuebler W. Diagnostic efficiency of troponin t measurements in acute myocardial infarction. Circulation. 1991;83:902-912
5. Morrow DA, Cannon CP, Jesse RL, Newby LK, Ravkilde J, Storrow AB, Wu AH, Christenson RH. National academy of clinical biochemistry laboratory medicine practice guidelines: Clinical characteristics and utilization of biochemical markers in acute coronary syndromes. Circulation. 2007;115:e356-375
6. Hoeller R, Rubini Gimenez M, Reichlin T, Twerenbold R, Zellweger C, Moehring B, Wildi K, Freese M, Stelzig C, Hartmann B, Stoll M, Mosimann T, Reiter M, Haaf P, Mueller M, Meller B, Hochgruber T, Balmelli C, Sou SM, Murray K, Freidank H, Steuer S, Minners J, Osswald S, Mueller C. Normal presenting levels of highsensitivity troponin and myocardial infarction. Heart. 2013
7. Baker JO, Reinhold J, Redwood S, Marber MS. Troponins: Redefining their limits. Heart. 2011;97:447-452
8. Jacquet S, Yin X, Sicard P, Clark J, Kanaganayagam GS, Mayr M, Marber MS. Identification of cardiac myosin-binding protein c as a candidate biomarker of myocardial infarction by proteomics analysis. Mol Cell Proteomics. 2009;8:2687-2699
9. Aye TT, Scholten A, Taouatas N, Varro A, Van Veen TA, Vos MA, Heck AJ. Proteome-wide protein concentrations in the human heart. Mol Biosyst. 2010;6:1917-1927
10. Sadayappan S, de Tombe PP. Cardiac myosin binding protein-c: Redefining its structure and function. Biophys Rev. 2012;4:93-106
11. Baker JO, Devaraj R, Reinhold J, Kanaganayagam G, Sadayappan S, Gautel M, Redwood S, Marber M. Cardiac myosin-binding protein c as a potential new serum biomarker of myocardial infarction. Circulation. 2010;122:A15438
12. Govindan S, Kuster DW, Lin B, Kahn DJ, Jeske WP, Walenga JM, Leya F, Hoppensteadt D, Fareed J, Sadayappan S. Increase in cardiac myosin binding protein-c plasma levels is a sensitive and cardiac-specific biomarker of myocardial infarction. Am J Cardiovasc Dis. 2013;3:60-70
13. Govindan S, McElligott A, Muthusamy S, Nair N, Barefield D, Martin JL, Gongora E, Greis KD, Luther PK, Winegrad S, Henderson KK, Sadayappan S. Cardiac myosin binding protein-c is a potential diagnostic biomarker for myocardial infarction. J Mol Cell Cardiol. 2012;52:154-164
14. Liebetrau C, Mollmann H, Nef H, Szardien S, Rixe J, Troidl C, Willmer M, Hoffmann J, Weber M, Rolf A, Hamm C. Release kinetics of cardiac biomarkers in patients undergoing trans-coronary ablation of septal hypertrophy. Clin Chem. 2012;58:1049-1054
15. Köhler, G., Milstein, C., Nature, 1975, 256 (5517): 495-497;
16. Kearney, J.F., Radbruch, A., Liesegang, B., Rajewsky, K., J. Immunol., 1979, 123(4): 1548 - 50;
17. Galfre, G., Milstein, C., Methods Enzymol., 1981, 73(Pt B): 3-46
18. Geysen, H.M., Rodda, S.J., Mason, T.J., Tribbick, G., Schoofs, P.G., J. Immunol. Methods, 1987,102(2): 259-274

## Patentansprüche

1. Hybridomzelllinie DSM ACC3224, die den monoklonalen Antikörper anti-My-C-1A4 gegen humanes, kardiales Myosin Binding Protein C produziert.

2. Monoklonaler Antikörper anti-My-C-1A4, **dadurch gekennzeichnet, dass** dieser das Epitop der Sequenz AELGESAPSPK im Bereich der Aminosäuren Alanin 125 bis Lysin 135 der Sequenz des humanen, kardialen My-C erkennt und bindet.

3. Monoklonaler Antikörper anti-My-C-1A4 nach Anspruch 2, **dadurch gekennzeichnet, dass** dieser von einer Hybridomzelllinie nach Anspruch 1 produziert wird.

4. Verfahren zur Herstellung monoklonaler Antikörper gemäß Anspruch 2 order 3, **dadurch gekennzeichnet, dass** eine Hybridomzelllinie gemäß Anspruch 1 kultiviert wird und die produzierten monoklonalen Antikörper isoliert werden.

5. In vitro Verwendung des monoklonalen Antikörpers gemäß Anspruch 2 und/oder 3 zu analytischen Zwecken, als Catcher- oder Detektor-Antikörper in einem ELISA zur Bestimmung der Konzentration von My-C im Serum oder Plasma und damit für die frühzeitige Diagnose von Myokardinfarkten.

6. In vitro Verwendung des monoklonalen Antikörpers gemäß Anspruch 2 und/oder 3 zu analytischen Zwecken, in Immunoblots, der Immunhistochemie und anderen analytischen in vitro Verfahren.

## Claims

1. Hybridoma cell line DSM ACC3224, which produces the monoclonal antibody anti-My-C-1A4 against human cardiac myosin binding protein C.

2. Monoclonal antibody anti-My-C-1A4, **characterized in that** it recognizes and binds the epitope of the sequence AELGESAPSPK in the region of the amino acids A125 to K135 of the sequence of the human cardiac My-C.

3. The monoclonal antibody anti-My-C-1A4 according to claim 2, **characterized in that** it is produced by a hybridoma cell line according to claim 1.

4. A method for producing monoclonal antibodies according to claim 2 or 3, **characterized in that** a hybridoma cell line according to claim 1 is cultured and the monoclonal antibodies produced are isolated.

5. In vitro use of the monoclonal antibody according to claim 2 and / or 3 for analytical purposes, in particular as catcher or detector antibody in an ELISA for determining the concentration of My-C in serum or plasma and hence for the early diagnosis of myocardial infarction.

6. In vitro use of the monoclonal antibody according to claim 2 and / or 3 for analytical purposes, in particular in immunoblots, immunohistochemistry and other in vitro analytical methods.

## Revendications

1. Lignée cellulaire d'hybridome DSM ACC3224, qui produit l'anticorps monoclonal anti-My-C-1A4 contre la protéine C liant la myosine cardiaque humaine.

2. Anticorps monoclonal anti-My-C-1A4, **caractérisé en ce qu'**il reconnaît et lie l'épitope de séquence AELGESAPSPK dans la région des acides aminés A125 à K135 de la séquence de la My-C cardiaque humaine.

3. Anticorps monoclonal anti-My-C-1A4 selon la revendication 2, **caractérisé en ce qu'**il est produit par une lignée cellulaire d'hybridome selon la revendication 1.

4. Procédé de production d'anticorps monoclonaux selon la revendication 2 ou 3, **caractérisé en ce qu'**une lignée cellulaire d'hybridome selon la revendication 1 est cultivée et les anticorps monoclonaux produits sont isolés.

5. Utilisation in vitro de l'anticorps monoclonal selon la revendication 2 et/ou 3 à des fins analytiques, en particulier comme anticorps capteur ou détecteur dans un ELISA pour déterminer la concentration de My-C dans le sérum ou le plasma et donc pour le diagnostic précoce d'infarctus du myocarde.

6. Utilisation in vitro de l'anticorps monoclonal selon la revendication 2 et/ou 3 à des fins analytiques, en particulier dans des immunotransferts, en immunohistochimie et d'autres procédés analytiques in vitro.
